# EUROPEAN PATENT SPECIFICATION

(11) **EP 1 853 270 B1**
(45) Date of publication and mention of the grant of the patent: **21.04.2010**
(21) Application number: 06708348.5
(22) Date of filing: 17.02.2006
(51) Int. Cl.: A61K 31/55, A61P 9/10, A61K 45/06, A61K 31/54

(54) **PHARMACEUTICAL COMPOSITIONS COMPRISING NEP-INHIBITORS, INHIBITORS OF THE ENDOGENOUS ENDOTHELIN PRODUCING SYSTEM AND DIURETICS**
PHARMAZEUTISCHE ZUSAMMENSETZUNGEN MIT NEP-INHIBITOREN, INHIBITOREN DES ENDOGENES ENDOTHELIN PRODUZIERENDEN SYSTEMS UND DIURETIKA
COMPOSITIONS PHARMACEUTIQUES COMPRENANT DES INHIBITEURS DE L'ENDOPEPTIDASE NEUTRE (EPN), DES INHIBITEURS DU SYSTEME DE PRODUCTION DE L ENDOTHELINE ENDOGENE AINSI QUE DES DIURETIQUES

(30) Priority: 18.02.2005 EP 05101235
(43) Date of publication of application: 14.11.2007
(73) Proprietor: Solvay Pharmaceuticals GmbH, 30173 Hannover (DE)
(72) Inventor: WITTE, Klaus, 30173 Hannover (DE); ZIEGLER, Dieter, 30966 Hemmingen (DE); STRAUB, Matthias, NL-1873 JW Groet (NL); FISCHER, Yvan, 30890 Barsinghausen (DE)
(74) Representative: Bauriegel, Lutz
(86) International application number: PCT/EP2006/060057
(87) International publication number: WO 2006/087371

(56) References cited:
- WO-A-02/094176
- WO-A-2004/082636
- WO-A-2005/030795
- US-A1- 2004 186 083
- US-A1- 2004 266 698
- SORBERA L A ET AL: "ANTIHYPERTENSIVE TREATMENT OF HEART FAILURE NEPRILYSIN INHIBITOR ENDOTHELIN-CONVERTING ENZYME INHIBITOR" DRUGS OF THE FUTURE, BARCELONA, ES, vol. 27, no. 1, 2002, pages 27-31, XP009026581 ISSN: 0377-8282
- O'CONNOR C M ET AL: "CURRENT AND NOVEL PHARMACOLOGIC APPROACHES IN ADVANCED HEART FAILURE" AMERICAN HEART JOURNAL, MOSBY- YEAR BOOK INC., ST. LOUIS, MO, US, vol. 135, no. 6, SUPPL II, June 1998 (1998-06), pages S249-S263, XP001004744 ISSN: 0002-8703
- GRAUL A I: "ANNUAL UPDATE 2003: CARDIOVASCULAR DRUGS" DRUGS OF THE FUTURE, BARCELONA, ES, vol. 28, no. 6, 1 June 2003 (2003-06-01), pages 565-625, XP009039185 ISSN: 0377-8282

## Description

The present invention relates to a novel combination therapy for cardiovascular, renal and/or further diseases or conditions, in particular for cardiovascular diseases involving hypertension, by administering a synergistic combination of daglutril and hydrochlorothiazide. Thus, the invention also relates to novel pharmaceutical compositions comprising daglutril and hydrochlorothiazide and the use of said pharmaceutical composition in the prophylaxis or treatment of cardiovascular, renal and/or further diseases in mammals and humans.

The nature of cardiovascular, in particular hypertensive vascular, diseases is multi-factorial. Combination therapy has been shown to address the multiple pathophysiologic factors that play a role in blood pressure elevation, including blood volume, vasoconstriction, and the impact of sympathetic nervous system and Renin-Angiotensin-Aldosterone-System (= RAAS) activity (see e.g. M.R. Weir, American Journal of Hypertension 11 (1998) 163S-169S), potentially resulting in both greater reduction in blood pressure and in lowered risks for target-organ damage. The use of a fixed, low-dose combination agent could also offer lower doses of each component than those that may be necessary with monotherapy, thus reducing the risks of dose-dependent adverse events and associated compliance problems.

From document EP 0 254 032 A2 (= US 4,749,688) it is known that NEP inhibitors can lower blood pressure under conditions where angiotensin converting enzyme (= ACE) inhibitors as a monotherapy are relatively ineffective.

In congestive heart failure, as a result of the decreased cardiac output and the increase in peripheral resistance, back-pressure phenomena of the blood occur in the pulmonary circulation and the heart itself. As a result, an increased wall tension of the heart muscle occurs in the area of the auricles and chambers. In such a situation, the heart functions as an endocrine organ and secretes, inter alia, the atrial natriuretic peptide (= ANP) into the bloodstream. Due to its marked vasodilatory and natriuretic/diuretic activity, ANP brings about both a reduction in the peripheral resistance and a decrease in the circulating blood volume. The consequence is a marked pre- and afterload decrease. This constitutes an endogenous cardioprotective mechanism. This positive endogenous mechanism is limited in that ANP has only a very short half-life in the plasma. The reason for this is that the hormone is very rapidly broken down by NEP. Therefore, pharmacological NEP inhibition rises ANP levels and thus promotes this cardioprotective mechanism. Due to a disease-related reduced output of the heart in congestive heart failure, a reflex increase in peripheral vascular resistance occurs. As a result, the heart muscle must begin to pump against an increased afterload. In a vicious cycle, this results in increased strain on the heart and worsens the situation further. The increase in the peripheral resistance is mediated, inter alia, by the vasoactive peptide endothelin. Endothelin (= ET) is the strongest presently known endogenous vasoconstrictory substance and is formed from the precursor big endothelin (= bigET) with participation of the endothelin converting enzyme (= ECE). Therefore, pharmacological inhibition lowers the levels of vasoconstrictive ET.

For these reasons, a combination of compounds having NEP-inhibiting activity with compounds capable of inhibiting the endogenous endothelin producing system or compounds with dual inhibiting activities on NEP and the endogenous endothelin producing system would seem to provide added value in the therapy of cardiovascular diseases like essential hypertension, pulmonary hypertension and/or congestive heart failure. As a result of inhibition of the endogenous endothelin producing system, formation of endothelin would be prevented and thus an increase in peripheral resistance would be counteracted, to result in a relief of the strain on the heart muscle. Inhibition of the ANP degrading enzyme NEP can thus lead to higher ANP levels and an increased duration of action of ANP. This will lead to a reinforcement of the ANP-mediated endogenous cardioprotective mechanism of action. However, because NEP may also be involved in ET degradation, a pure NEP inhibition would, in addition to the desired increase in the ANP levels, also lead to an unfavorable increase in the ET levels. For this reason, a mixed profile with dually acting inhibition of NEP and of the endogenous endothelin producing system is to be regarded as particularly favorable, since it prevents both the breakdown of the natriuretically/diuretically acting ANP (by NEP-blockade), and simultaneously inhibits the formation of ET. As a result, the adverse attendant effect of pure NEP-inhibitors (increase in the endothelin levels) no longer comes to bear.

Compounds with a dually acting combined inhibitory effect on NEP and the endogenous endothelin producing system, i.e. benzazepine-, benzoxazepine- and benzothiazepine-N-acetic acid derivatives, are known from document EP 0 733 642 A1 (= US 5,677,297). Further favourable pharmacological properties of compounds falling within the structural scope of EP 0 733 642 A1 are known from documents EP 0 830 863 A1 (= US 5,783,573), WO 00/48601 A1 (= US 6,482,820) and WO 01/03699 A1 (=US-2003-0040512-A1).

Phosphonic acid substituted benzazepinone-N-acidic acid derivatives with a combined inhibitory effect on NEP and the endogenous endothelin producing system are disclosed in document EP 0 916 679 A1 (= US 5,952,327).

Amidomethyl-substituted 1-(carboxyalkyl)-cyclopentylcarbonylamino-benzazepine-N-acetic acid derivatives which are useful e.g. for the prophylaxis and/or treatment of cardiovascular conditions or diseases, are disclosed in document WO 2005/030795 A1.

From document WO 02/094176 A2 it is known that certain compounds, including those disclosed in document EP 0 733 642 A1 and in document EP 0 916 679 A1, may inhibit the endogenous endothelin producing system *via* an inhibition of metalloprotease IGS5. The metalloprotease IGS5 is also known as human soluble endopeptidase (= hSEP) and is described e.g. in document WO 02/094176 A2. Further, WO 02/094176 A2 discloses the use of compounds with combined NEP/hSEP inhibitory activity for the prophylaxis or treatment of *inter alia* cardiovascular diseases.

Diuretics are drugs which act on the kidney to promote excretion of water and electrolytes, particularly sodium. These drugs are e.g. widely used in treating edematous conditions such as those associated with cardiovascular diseases. Certain combinations of cardiovascular active agents with diuretics are already known.

In international patent application published as WO 2004/082636, a combination of an aldosterone receptor antagonist and a neutral endopeptidase inhibitor is disclosed.

Published US patent application No. 2004/0186083 provides a combination of an aldosterone receptor antagonist and an endothelin receptor antagonist and/or endothelin converting enzyme inhibitor.

Published US patent application No. 2004/0266698 discloses pyrane derivatives as both ACE- and NEP inhibitors.

International patent application WO 2006/000564 refers to pharmaceutical compositions comprising NEP-inhibitors, inhibitors of the endogenous endothelin producing system and AT, receptor antagonists.

It is the object of the present invention to provide a novel combination therapy for cardiovascular diseases, renal diseases and/or further diseases with enhanced efficacy and a favourable safety profile.

It has now surprisingly been found that a combination of daglutril and additionally hydrochlorothiazide, provides still further enhanced efficacy and a favourable safety profile in the prophylaxis or treatment of cardiovascular diseases, renal diseases and/or further diseases.

The invention therefore relates in a first aspect to pharmaceutical compositions comprising
i) as active agent a pharmacologically effective quantity of
   (a) at least one neutral endopeptidase-inhibitor,
   (b) at least one inhibitor of the endogenous endothelin producing system and
   (c) at least one diuretic which is hydrochlorothiazide or any physiologically compatible tautomer, salt, solvate or ester thereof,
   wherein the combination of at least one neutral endopeptidase inhibitor (a) and at least one inhibitor of the endogenous endothelin producing system (b) is a dually acting compound capable of inhibiting neutral endopeptidase and human soluble endopeptidase, which is daglutril and/or physiologically compatible salts of daglutril, and
ii) optionally conventional pharmaceutically acceptable auxiliaries and/or carriers.

The pharmaceutical compositions according to the invention may preferably comprise conventional pharmaceutically acceptable auxiliaries and/or carriers.

In the pharmaceutical compositions according to the invention, the subcombination of at least one NEP-inhibitor a) and at least one inhibitor of the endogenous endothelin producing system b) is realized by a dually acting compound which is daglutril and/or physiologically compatible salts of acids of daglutril.

Suitable physiologically compatible salts of daglutril include its alkali metal, alkaline earth metal or ammonium salts, for example sodium or calcium salts or salts with physiologically compatible, pharmacologically neutral organic amines such as, for example, diethylamine or tert.-butylamine.

The compound of Formula II, 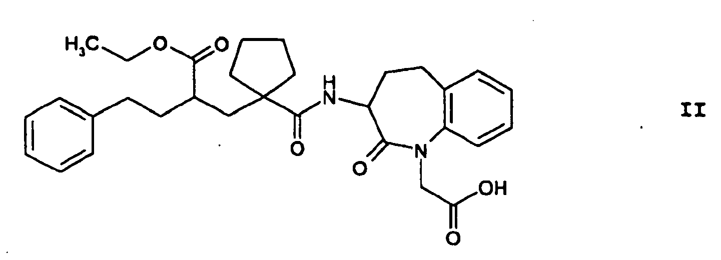 in its 3S,2'R form is "daglutril" or "SLV306". Daglutril is known, for example, from document EP 0 733 642 A1 or patent US 5,677,297 and can be produced according to the production processes disclosed or referenced in this document or analogously to said production processes.

The diuretic which can be used according to the present invention is understood to comprise any physiologically compatible salt, solvate or ester thereof and is a thiazide diuretic.

The combination of daglutril and additionally hydrochlorothiazide according to the invention has been found to provide still further enhanced efficacy and a favourable safety profile in the prophylaxis or treatment of cardiovascular diseases, renal diseases and/or further diseases.

Cardiocvascular diseases according to the invention may e.g. comprise acute coronary syndrome; acute heart failure; angina pectoris; angina abdominalis; arrhythmias; cardiac hypertrophy; cerebral infarction; cerebral ischemias; chronic heart failure; congestive heart failure; coronary heart disease; critical leg ischemia; hypertension, in particular essential hypertension, pulmonary hypertension, renal hypertension and/or hypertension associated with obesity, insulin resistance and/or diabetes; myocardial infarction; restenosis and/or stroke. The use of the combinations according to the invention in treating hypertension in its different forms and from different origins is preferred.

Renal diseases according to the invention may be of different origins, and may e.g. comprise renal diseases due to heart failure, diabetes or toxic agents. More specifically, renal diseases may comprise e.g. acute renal failure; chronic renal failure (chronic kidney disease), in particular diabetic nephropathy; and/or ischemic renal failure. Renal diseases due to toxic substances may e.g. be due to the prior administration of nephrotoxic radiocontrast media or cyclosporin. Cardiovascular diseases and renal diseases may occur jointly.

Further diseases according to the invention may e.g. comprise liver fibrosis and/or liver cirrhosis.

The pharmaceutical compositions according to the invention can be prepared in a manner known per se and thus can be obtained as formulations suitable for enteral, such as oral or rectal, or parenteral administration to mammals or humans, comprising a therapeutical effective amount of the pharmacologically active agents, alone or in combination with one or more pharmaceutically acceptable auxiliaries and/or carriers, especially suitable for enteral or parenteral application. Pharmaceutical compositions for enteral or parenteral administration are, for example, in unit dosage forms, such as coated tablets, tablets, capsules or suppositories and also ampoules. These are prepared in a manner which is known per se, for example using conventional mixing, granulation, coating, solubulizing or lyophilizing processes. Typical oral formulations include coated tablets, tablets, capsules, syrups, elixirs and suspensions. Capsules may contain the active agents e.g. in form of powders, granules, pellets, beadlets or microtablets. For example, a pharmaceutical composition according to the invention may consist of from about 0.1 % to 90%, preferably of from about 1 % to about 80%, of the active agents, the rest being made up by pharmaceutically acceptable auxiliaries and/or carriers. Thus, pharmaceutical compositions for oral use can be obtained by combining the active compounds with solid excipients, if desired granulating a mixture which has been obtained, and, if required or necessary, processing the mixture or granulate into tablets or coated tablet cores after having added suitable auxiliary substances. Typical injectable formulations include solutions and suspensions.

In one embodiment of the pharmaceutical compositions according to the invention, the active agents can be obtained and administered together, e.g. in one combined unit dosage form like in one tablet or capsule, i.e. in a physical combination. In such a combined unit dosage form, the different active agents can be segregated from each other, e.g. by means of different layers in said tablet, e.g. by the use of inert intermediate layers known in the art; or by means of different compartments in said capsule. The active agents [(a) + (b)] and (c) in the pharmaceutical composition can favourably be present in two separate dosage forms, usually complementary or balanced for combined use, e.g. as two different tablets or capsules, usually further comprising pharmaceutically acceptable auxiliaries and/or carriers, or in different compartments of one single capsule. Thus, in this embodiment at least the diuretic is present in a unit single dosage form physically segregated from the other active agent. The corresponding active agents or their pharmaceutically acceptable salts may also be used in form of their hydrates or include other solvents used for crystallization. A unit dosage form may be a fixed combination. A unit dosage form, in particular a fixed combination of the active agents is a preferred alternative of this embodiment.

In another embodiment the active agents can be obtained and administered in two or more separate unit dosage forms, e.g. in two tablets or capsules, the tablets or capsules being physically segregated from each other. The two separate unit dosage forms can be administered simultaneously or stepwise (separately), e.g. sequentially one after the other in either order. Thus, the active agents can be administered in either order at the same time or at different times spread over the day, the optimal dosage regimen usually being determined by prescription of a physician.

The typical pharmaceutically acceptable auxiliaries and/or carriers for use in the formulations described above are exemplified by: sugars such as lactose, sucrose, mannitol and sorbitol; starches such as cornstarch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone; polyvinyl alcohol; stearic acid; alkaline earth metal stearates such as magnesium stearate and calcium stearate; stearic acid; vegetable oils such as peanut oil, cottonseed oil, sesame oil, olive oil and corn oil; non-ionic, cationic and anionic surfactants; ethylene glycol polymers; betacyclodextrin; fatty alcohols; and hydrolyzed cereal solids, as well as other non-toxic compatible fillers, binders, disintegrants, agents, e.g. talcum; buffers, preservatives, antioxidants, lubricants, flavoring and the like commonly used in pharmaceutical formulations.

In a specific embodiment of said first aspect, the invention also relates to a kit comprising in separate containers in a single package pharmaceutical dosage forms for use in combination, comprising,
i) in one separate container a pharmaceutical dosage form comprising a dually acting compound capable of inhibiting neutral endopeptidase and the endogenous endothelin producing system which is daglutril, and
ii) in another separate container a pharmaceutical dosage form comprising a diuretic, which is hydrochlorothiazide.

The kit form is particularly advantageous but not limited to the case when the separate components must be administered in different dosage forms or are administered at different dosage intervals. The dosage forms may favourably be oral formulations like tablets or capsules. The separate containers may e.g. be blister packs (in particular where the oral formulations are tablets or coated tablets), boxes or other containers commonly used to package pharmaceutical dosage forms. Preferred are alternatives of the kit which comprise in one separate container a pharmaceutical dosage form comprising daglutril or any of its physiologically compatible salts or esters; and in another separate container a pharmaceutical dosage form comprising hydochlorothiazide or any of its physiologically compatible salts.

In a second aspect, the invention also relates to a use of daglutril and hydrochlorothiazide, for the preparation of a pharmaceutical composition or medicament for the prophylaxis or treatment of cardiovascular diseases, renal diseases and/or further diseases as discussed in more detail above.

In one specific embodiment, a fixed combination of daglutril and hydrochlorothiazide can be used.

### Description of the test method

The beneficial effects of the combination therapy according to the invention can be shown in suitable pharmacological test models, e.g. in the in vivo test model as laid down in more detail below, or in closely related test models:

Male spontaneously hypertensive rats (= SHR, insulin resistant strain from Charles River; aged 6 months) were equipped with telemetry transmitters for continuous monitoring of blood pressure and heart rate (TA11PA-C40. DSI, USA). Telemetry transmitters for continuous monitoring of blood pressure, heart rate and locomotor activity (TA11PA-C40, Data Sciences, USA) were implanted intraabdominally under inhalative halothane anesthesia. A midline abdominal incision was made, and the abdominal aorta was visualized by removal of retroperitoneal fat and connective tissue. A ligature was placed caudal of the renal arteries, the aorta was punctured with a 22G needle, and the catheter was advanced into the aorta. The entry point was sealed with tissue adhesive (Vetbond, 3M, USA), the ligature was removed, and the abdominal incision was closed. Measurements of aortic pressure were taken every 5 minutes (= min) for 4 seconds (= s) each at a sampling rate of 500 Hz, and were corrected for the corresponding ambient pressure (ambient pressure monitor, C11 PR, Data Sciences, USA).

After 3 days of monitoring under baseline (untreated) conditions, animals received daglutril via the drinking water. The intended daily dose was 100 mg/kg/day of daglutril. The concentration in drinking water was adjusted once per week, resulting in an average drug intake of 98 mg/kg/day.

In a second experiment, rats were divided into two treatment groups receiving hydrochlorothiazide or hydrochlorothiazide + daglutril. Compounds were administered via the drinking water, and daily drug intake was measured by weighing the water bottles thrice weekly. Intended daily doses were 10 mg/kg/day of hydrochlorothiazide plus, in the combination group, 100 mg/kg/day of daglutril.

Concentrations of hydrochlorothiazide and daglutril in the drinking water were adjusted once per week, in order to ensure the intended daily intake of 10 and 100 mg/kg, respectively. The average daily water intake amounted to 57 and 46 ml/kg in the hydrochlorothiazide and hydrochlorothiazide + daglutril group, respectively, resulting in the uptake of 9.5 mg/kg/day of hydrochlorothiazide in the hydrochlorothiazide group, and 9.4 mg/kg/day of hydrochlorothiazide and 94.4 mg/kg/day of daglutril in the combination group.

The blood pressure, heart rate and activity values, sampled in 5 min intervals by the Dataquest^{™} system, were used for calculation of individual 24 hours (= h) -means. These 24 h means were exported to Excel, and group mean values of systolic blood pressure (= SBP), diastolic blood pressure (= DBP), heart rate (= HR), and locomotor activity (= ACT) were calculated for the daglutril, hydrochlorothiazide and the hydrochlorothiazide + daglutril groups. For the statistical analysis, a baseline value (pre) was calculated from the last day prior to compound application (day 3), and effects of daglutril, hydrochlorothiazide and hydrochlorothiazide + daglutril were calculated in relation to this baseline value (day 23, i.e. after 3 weeks of treatment, minus baseline value). The statistical comparison was done by using univariate ANOVA at an error level of P<0.05.

In this test model, administration of daglutril in combination with a thiazide diuretic (hydrochlorothiazide) and compared to administration of the diuretic hydrochlorothiazide only and daglutril only, showed the results as given in table 1 below:

**Table 1: Effects of coadministration of daglutril and a thiazide diuretic (hydrochlorothiazide) on cardiovascular parameters in the spontaneously hypertensive rat**

| **CV** | **HCTZ only** | | **daglutril only** | | **HCTZ + daglutril** | | **statistics** |
|---|---|---|---|---|---|---|---|
| **parameters** | Mean | SEM | Mean | SEM | Mean | SEM | ANOVA |
| DBP [mmHg] | -7.7 | 0.6 | 2.0 | 0.5 | -9.3 ^{(*)} | 0.5 | P<0.001 |
| SBP [mmHg] | -11.3 | 0.7 | -0.3 | 0.9 | -14.9* | 0.7 | P<0.001 |
| HR [1/min] | -4.5 | 1.7 | -6.0 | 2.1 | -7.7 | 2.4 | n.s. |

| | | | | | | | |
|---|---|---|---|---|---|---|---|
| HCTZ= hydrochlorothiazide; n=5 animals per group; SEM= Standard Error of the Mean, two-tailed ANOVA, n.s. = not significant, ^{(*)} P<0.1, * P<0.01 two-tailed t-test HCTZ versus HCTZ + SLV306 | | | | | | | |

In this test model, hydrochlorothiazide only resulted in a moderate decrease in blood pressure, daglutril only had no effect on blood pressure, while the combination group hydrochlorothiazide + daglutril showed a significantly greater decrease in blood pressure than either of the monotherapy groups. The difference in blood pressure effects between the groups was statistically significant (ANOVA, at least P<0.01).

The dosage of the active agents can depend on a variety of factors, such as mode of administration, species, age and/or individual condition. Suitable dosages for the active agents of the pharmaceutical combination according to the present invention are therapeutically effective dosages, for example those which are commercially available. Normally, in the case of oral administration, an approximate daily dose of from about 3 mg to about 2000 mg is to be estimated for each of the active agents e.g. for a patient of approximately 75 kg in weight. For example, a pharmaceutical composition according to the invention may preferably comprise daglutril as dually acting compound capable of inhibiting NEP and the endogenous endothelin producing system in the range of 50-800 mg, preferably in the range of 200-600 mg. The daily dose range of the diuretic which can be used in the pharmaceutical compositions according to the invention may vary and may for example be (calculated for the pure active substance, not the salt or solvate thereof), 200 mg for hydrochlorothiazide. The administration of the pharmaceutical composition may occur up to three times a day. Once daily administration forms are preferred.

### Example I:

### Capsules containing daglutril and hydrochlorothiazide:

Capsules with the following composition per capsule were produced:

| | |
|---|---|
| Daglutril calcium salt | 250 mg |
| Hydrochlorothiazide | 50 mg |
| Com starch | 50 mg |
| Lactose | 30 mg |
| Ethyl acetate | q.s. |

The active agents, the com starch and the lactose were processed into a homogeneous pasty mixture using ethyl acetate. The paste was ground and the resulting granules were placed on a suitable tray and dried at 45°C in order to remove the solvent. The dried granules were passed through a crusher and mixed in a mixer with the further following auxiliaries:

| | |
|---|---|
| Talcum | 5 mg |
| Magnesium stearate | 5 mg |
| Com starch | 10 mg |

and then poured into 400 mg capsules (= capsule size 0).

## Claims

1. A pharmaceutical composition comprising
i) as active agent a pharmacologically effective quantity of
(a) at least one neutral endopeptidase-inhibitor,
(b) at least one inhibitor of the endogenous endothelin producing system and
(c) at least one diuretic which is hydrochlorothiazide or any physiologically compatible tautomer, salt, solvate or ester thereof,
wherein the combination of at least one neutral endopeptidase inhibitor (a) and at least one inhibitor of the endogenous endothelin producing system (b) is a dually acting compound capable of inhibiting neutral endopeptidase and human soluble endopeptidase, which is daglutril and/or physiologically compatible salts of daglutril, and
ii) optionally conventional pharmaceutically acceptable auxiliaries and/or carriers.

2. Pharmaceutical composition according to claim 1, comprising conventional pharmaceutically acceptable auxiliaries and/or carriers.

3. Pharmaceutical composition according to claim 1 which is suitable for oral administration.

4. Pharmaceutical composition according to claim 3 wherein the active agents are present in one or more dosage forms selected from the group consisting of tablets, coated tablets, capsules, syrups, elixirs or suspensions.

5. Pharmaceutical composition according to claim 1, wherein the diuretic is present in a unit single dosage form physically segregated from the neutral endopeptidase-inhibitor and/or the inhibitor of the endogenous endothelin producing system.

6. A use of at least one neutral endopeptidase inhibitor in combination with at least one inhibitor of the endogenous endothelin producing system, which is the dually acting compound daglutril and/or physiologically compatible salts of daglutril, which is capable of inhibiting neutral endopeptidase and human soluble endopeptidase, and at least one diuretic which is hydrochlorothiazide or any physiologically compatible tautomer, salt, solvate or ester thereof, for the manufacture of a medicament for the prophylaxis or treatment of a cardiovascular disease and/or a renal disease in mammals and humans.

7. Use according to claim 6, wherein the cardiovascular disease is selected from the group consisting of acute coronary syndrome; acute heart failure; angina pectoris; angina abdominalis; arrhythmias; cardiac hypertrophy; cerebral infarction; cerebral ischemias; chronic heart failure; congestive heart failure; coronary heart disease; critical leg ischemia; hypertension, in particular essential hypertension, pulmonary hypertension, renal hypertension and/or hypertension associated with obesity, insulin resistance and/or diabetes; myocardial infarction; restenosis and/or stroke.

8. Use according to claim 6, wherein the renal disease is selected from the group consisting of acute renal failure; chronic renal failure, in particular diabetic nephropathy; ischemic renal failure; and/or renal diseases due to toxic substances.

9. Use according to claim 6, wherein the neutral endopeptidase inhibitor, the inhibitor of the endogenous endothelin producing system and the diuretic are arranged for simultaneous or stepwise (separate) use or for use in physical combination.

10. Use according to claim 6, wherein a fixed combination of the neutral endopeptidase inhibitor, the inhibitor of the endogenous endothelin producing system and the diuretic is used.

11. Use according to claim 6 wherein the dually acting compound capable of inhibiting neutral endopeptidase and human soluble endopeptidase, and the diuretic are administered simultaneously, stepwise (separately) or in physical combination.

12. A combination of daglutril and/or physiologically compatible salts of daglutril, and hydrochlorothiazide or any physiologically compatible tautomer, salt, solvate or ester thereof, for the prophylaxis or treatment of a cardiovascular disease and/or a renal disease in mammals and humans.

13. A kit comprising in separate containers in a single package pharmaceutical dosage forms for use in combination, comprising,
i) in one separate container a pharmaceutical dosage form comprising a dually acting compound capable of inhibiting neutral endopeptidase and the endogenous endothelin producing system which is daglutril and/or physiologically compatible salts of daglutril, and
ii) in another separate container a pharmaceutical dosage form comprising a diuretic which is hydrochlorothiazide or any physiologically compatible tautomer, salt, solvate or ester thereof,
the pharmaceutical dosage forms being suitable for simultaneous, separate or step-wise administration.

14. Kit according to claim 13, comprising in separate containers in a single package pharmaceutical dosage forms for use in combination, comprising,
i) in one separate container a pharmaceutical dosage form comprising a dually acting compound capable of inhibiting neutral endopeptidase and the endogenous endothelin producing system which is daglutril and/or physiologically compatible salts of daglutril, and
ii) in another separate container a pharmaceutical dosage form comprising a diuretic which is hydrochlorothiazide or any physiologically compatible tautomer, salt, solvate or ester thereof.

15. Kit according to claim 13 or 14, further comprising a leaflet indicating that the dually acting compound capable of inhibiting neutral endopeptidase and the endogenous endothelin producing system may be administered in combination with the diuretic simultaneously, step-wise (separately) or in physical combination.

## Patentansprüche

1. Pharmazeutische Zusammensetzung, enthaltend,
i) als Wirkstoff, eine pharmakologisch wirksame Menge
(a) wenigstens eines Inhibitors der neutralen Endopeptidase,
(b) wenigstens eines Inhibitors des endogenen Endothelin produzierenden Systems und
(c) wenigstens eines Diuretikums, bei dem es sich um Hydrochlorothiazid oder ein physiologisch kompatibles Tautomer, Salz oder Solvat oder einen physiologisch kompatiblen Ester davon handelt,
wobei die Kombination von wenigstens einem Inhibitor der neutralen Endopeptidase (a) und wenigstens einem Inhibitor des endogenen Endothelin produzierenden Systems (b) eine dual wirkende Verbindung ist, die dazu in der Lage ist, neutrale Endopeptidase und humane lösliche Endopeptidase zu inhibieren, bei der es sich um Daglutril und/oder physiologisch kompatible Salze von Daglutril handelt, und
ii) gegebenenfalls herkömmliche pharmazeutische unbedenkliche Hilfsstoffe und/oder Träger.

2. Pharmazeutische Zusammensetzung nach Anspruch 1, enthaltend herkömmliche pharmazeutisch unbedenkliche Hilfsstoffe und/oder Träger.

3. Pharmazeutische Zusammensetzung nach Anspruch 1, die für die orale Verabreichung geeignet ist.

4. Pharmazeutische Zusammensetzung nach Anspruch 3, wobei die Wirkstoffe in einer oder mehreren Verabreichungsformen ausgewählt aus der aus Tabletten, beschichteten Tabletten, Kapseln, Sirupen, Elixieren und Suspensionen bestehenden Gruppe vorliegen.

5. Pharmazeutische Zusammensetzung nach Anspruch 1, wobei das Diuretikum in einer Einheitseinzeldosisform vorliegt, die physikalisch getrennt von dem Inhibitor der neutralen Endopeptidase und/oder dem Inhibitor des endogenen Endothelin produzierenden Systems ist.

6. Verwendung wenigstens eines Inhibitors der neutralen Endopeptidase in Kombination mit wenigstens einem Inhibitor des endogenen Endothelin produzierenden Systems, bei dem es sich um die dual wirkende Verbindung Daglutril und/oder physiologisch kompatible Salze von Daglutril handelt, das/die dazu in der Lage ist/sind, neutrale Endopeptidase und humane lösliche Endopeptidase zu inhibieren, und wenigstens einem Diuretikum, bei dem es sich um Hydrochlorothiazid oder ein physiologisch kompatibles Tautomer, Salz oder Solvat oder einen physiologisch kompatiblen Ester davon handelt, zur Herstellung eines Medikaments zur Prophylaxe oder Behandlung einer Herz-Kreislauf-Krankheit und/oder einer Nierenkrankheit in Säugetieren und Menschen.

7. Verwendung nach Anspruch 6, wobei die Herz-Kreislauf-Krankheit aus der aus akutem Koronarsyndrom, akuter Herzinsuffizienz, Angina pectoris, Angina abdominalis, Arrhythmien, kardialer Hypertrophie, Hirninfarkt, zerebralen Ischämien, chronischer Herzinsuffizienz, dekompensierter Herzinsuffizienz, koronarer Herzkrankheit, kritischer Beinischämie, Hypertonie, insbesondere essentieller Hypertonie, pulmonaler Hypertonie, renaler Hypertonie und mit Obesitas, Insulinresistenz und/oder Diabetes assoziierte Hypertonie, Herzinfarkt, Restenose und/oder Schlaganfall bestehenden Gruppe ausgewählt ist.

8. Verwendung nach Anspruch 6, wobei die Nierenkrankheit aus der aus akutem Nierenversagen, chronischem Nierenversagen, insbesondere diabetischer Nephropathie, ischämischem Nierenversagen und/oder Nierenkrankheiten, die auf toxische Substanzen zurückzuführen sind, bestehenden Gruppe ausgewählt ist.

9. Verwendung nach Anspruch 6, wobei der Inhibitor der neutralen Endopeptidase, der Inhibitor des endogenen Endothelin produzierenden Systems und das Diuretikum für eine gleichzeitige oder schrittweise (getrennte) Verwendung oder für eine Verwendung in einer physikalischen Kombination ausgelegt sind.

10. Verwendung nach Anspruch 6, wobei eine feste Kombination des Inhibitors der neutralen Endopeptidase, des Inhibitors des endogenen Endothelin produzierenden Systems und des Diuretikums verwendet wird.

11. Verwendung nach Anspruch 6, wobei die dual wirkende Verbindung, die dazu in der Lage ist, neutrale Endopeptidase und humane lösliche Endopeptidase zu inhibieren, und das Diuretikum gleichzeitig, schrittweise (getrennt) oder in einer physikalischen Kombination verabreicht werden.

12. Kombination von Daglutril und/oder physiologisch kompatiblen Salzen von Daglutril und Hydrochlorothiazid oder einem physiologisch kompatiblen Tautomer, Salz, Solvat oder Ester davon zur Prophylaxe oder Behandlung einer Herz-Kreiskreislauf-Krankheit und/oder einer Nierenkrankheit in Säugetieren und Menschen.

13. Kit, enthaltend, in getrennten Behältnissen in einer einzelnen Packung, pharmazeutische Verabreichungsformen zur kombinierten Verwendung, umfassend
i) in einem getrennten Behältnis eine pharmazeutische Verabreichungsform, die eine dual wirkende Verbindung umfasst, die dazu in der Lage ist, neutrale Endopeptidase und das endogene Endothelin produzierende System zu inhibieren, bei der es sich um Daglutril und/oder physiologisch kompatible Salze von Daglutril handelt, und
ii) in einem anderen getrennten Behältnis eine pharmazeutische Dosierungsform, die ein Diuretikum umfasst, bei dem es sich um Hydrochlorothiazid oder ein physiologisch kompatibles Tautomer, Salz oder Solvat oder einen physiologisch kompatiblen Ester davon handelt,
wobei die pharmazeutischen Verabreichungsformen für die gleichzeitige, getrennte oder schrittweise Verabreichung geeignet sind.

14. Kit nach Anspruch 13, umfassend, in getrennten Behältnissen in einer einzelnen Packung, pharmazeutische Verabreichungsformen zur kombinierten Verwendung, umfassend
i) in einem getrennten Behältnis eine pharmazeutische Verabreichungsform, die eine dual wirkende Verbindung umfasst, die dazu in der Lage ist, neutrale Endopeptidase und das endogene Endothelin produzierende System zu inhibieren, bei der es sich um Daglutril und/oder physiologisch kompatible Salze von Daglutril handelt, und
ii) in einem anderen getrennten Behältnis eine pharmazeutische Dosierungsform, die ein Diuretikum umfasst, bei dem es sich um Hydrochlorothiazid oder ein physiologisch kompatibles Tautomer, Salz oder Solvat oder einen physiologisch kompatiblen Ester davon handelt.

15. Kit nach Anspruch 13 oder 14, weiterhin umfassend einen Beipackzettel, aus dem hervorgeht, dass die dual wirkende Verbindung, die dazu in der Lage ist, neutrale Endopeptidase und das endogene Endothelin produzierende System zu inhibieren, in Kombination mit dem Diuretikum gleichzeitig, schrittweise (getrennt) oder in physikalischer Kombination verabreicht werden kann.

## Revendications

1. Composition pharmaceutique comprenant
i) comme agent actif une quantité pharmacologiquement efficace de
(a) au moins un inhibiteur de l'endopeptidase neutre ;
(b) au moins un inhibiteur du système produisant de l'endothéline endogène ; et
(c) au moins un diurétique qui est l'hydrochlorothiazide ou n'importe quel tautomère, sel, solvate ou ester physiologiquement compatible de celui-ci,
l'association d'au moins un inhibiteur de l'endopeptidase neutre (a) et d'au moins un inhibiteur du système produisant de l'endothéline endogène (b) étant un composé à double action apte à inhiber l'endopeptidase neutre et l'endopeptidase humaine soluble, qui est le daglutril et/ou des sels physiologiquement compatibles du daglutril, et
ii) facultativement des adjuvants et/ou véhicules pharmaceutiquement acceptables classiques.

2. Composition pharmaceutique selon la revendication 1, comprenant des adjuvants et/ou véhicules pharmaceutiquement acceptables classiques.

3. Composition pharmaceutique selon la revendication 1 qui est appropriée pour une administration orale.

4. Composition pharmaceutique selon la revendication 3 dans laquelle les agents actifs sont présents sous une ou plusieurs formes de dosage choisies dans le groupe constitué par les comprimés, les comprimés enrobés, les capsules, les sirops, les élixirs ou les suspensions.

5. Composition pharmaceutique selon la revendication 1, dans laquelle le diurétique est présent sous une forme de dosage unitaire unique physiquement séparée de l'inhibiteur de l'endopeptidase neutre et/ou de l'inhibiteur du système produisant de l'endothéline endogène.

6. Utilisation d'au moins un inhibiteur de l'endopeptidase neutre en association avec au moins un inhibiteur du système produisant de l'endothéline endogène, qui est le composé à double action daglutril et/ou des sels physiologiquement compatibles du daglutril, qui est apte à inhiber l'endopeptidase neutre et l'endopeptidase humaine soluble, et d'au moins un diurétique qui est l'hydrochlorothiazide ou n'importe quel tautomère, sel, solvate ou ester physiologiquement compatible de celui-ci, pour la fabrication d'un médicament pour la prophylaxie ou le traitement d'une maladie cardiovasculaire et/ou d'une maladie rénale chez l'homme et les mammifères.

7. Utilisation selon la revendication 6, la maladie cardiovasculaire étant choisie dans le groupe constitué par le syndrome coronarien aigu ; l'insuffisance cardiaque aiguë ; l'angine de poitrine ; l'infarctus mésentérique ; les arythmies cardiaques ; l'hypertrophie cardiaque ; l'infarctus cérébral ; les ischémies cérébrales ; l'insuffisance cardiaque chronique ; l'insuffisance cardiaque congestive ; une maladie cardiaque coronarienne ; l'ischémie critique des membres inférieurs ; l'hypertension artérielle, en particulier l'hypertension artérielle essentielle, l'hypertension artérielle pulmonaire, l'hypertension artérielle rénale et/ou l'hypertension artérielle associée à l'obésité, la résistance à l'insuline et/ou le diabète ; l'infarctus du myocarde ; la resténose et/ou l'accident vasculaire cérébral.

8. Utilisation selon la revendication 6, la maladie rénale étant choisie dans le groupe constitué par l'insuffisance rénale aiguë ; l'insuffisance rénale chronique, en particulier la néphropathie diabétique ; l'insuffisance rénale ischémique ; et/ou les maladies rénales dues à des substances toxiques.

9. Utilisation selon la revendication 6, dans laquelle l'inhibiteur de l'endopeptidase neutre, l'inhibiteur du système produisant de l'endothéline endogène et le diurétique sont conçus pour une utilisation simultanée ou séquentielle (séparée) ou pour une utilisation en association physique.

10. Utilisation selon la revendication 6, dans laquelle une association fixée de l'inhibiteur de l'endopeptidase neutre, de l'inhibiteur du système produisant de l'endothéline endogène et du diurétique est utilisée.

11. Utilisation selon la revendication 6 dans laquelle le composé à double action apte à inhiber l'endopeptidase neutre et l'endopeptidase humaine soluble et le diurétique sont administrés simultanément, séquentiellement (séparément) ou en association physique.

12. Association de daglutril et/ou de sels physiologiquement compatibles du daglutril et d'hydrochlorothiazide ou de n'importe quel tautomère, sel, solvate ou ester physiologiquement compatible de celui-ci, pour la prophylaxie ou le traitement d'une maladie cardiovasculaire et/ou d'une maladie rénale chez l'homme et les mammifères.

13. Trousse comprenant dans des récipients séparés dans un seul emballage des formes pharmaceutiques de dosage destinées à être utilisées en association, comprenant,
i) dans un récipient séparé une forme pharmaceutique de dosage comprenant un composé à double action apte à inhiber l'endopeptidase neutre et le système produisant de l'endothéline endogène qui est le daglutril et/ou des sels physiologiquement compatibles du daglutril et
ii) dans un autre récipient séparé une forme pharmaceutique de dosage comprenant un diurétique qui est l'hydrochlorothiazide ou n'importe quel tautomère, sel, solvate ou ester physiologiquement compatible de celui-ci,
les formes pharmaceutiques de dosage étant appropriées pour une administration simultanée, séparée ou séquentielle.

14. Trousse selon la revendication 13, comprenant dans des récipients séparés dans un seul emballage des formes pharmaceutiques de dosage destinées à être utilisées en association, comprenant,
i) dans un récipient séparé une forme pharmaceutique de dosage comprenant un composé à double action apte à inhiber l'endopeptidase neutre et le système produisant de l'endothéline endogène qui est le daglutril et/ou des sels physiologiquement compatibles du daglutril et
ii) dans un autre récipient séparé une forme pharmaceutique de dosage comprenant un diurétique qui est l'hydrochlorothiazide ou n'importe quel tautomère, sel, solvate ou ester physiologiquement compatible de celui-ci.

15. Trousse selon la revendication 13 ou 14, comprenant en outre une notice indiquant que le composé à double action apte à inhiber l'endopeptidase neutre et le système produisant de l'endothéline endogène peut être administré en association avec le diurétique simultanément, séquentiellement (séparément) ou en association physique.
